# EUROPÄISCHE PATENTANMELDUNG

(11) **EP 2 008 565 A2**
(43) Veröffentlichungstag der Anmeldung: **31.12.2008**
(21) Anmeldenummer: 08010155.3
(22) Anmeldetag: 04.06.2008
(51) Int. Cl.: A47L 13/16, A47L 13/20, D04H 1/60, D04H 1/64, D04H 1/42, D04H 13/00

(54) **Reinigungsartikel und dessen Verwendung**

(30) Priorität: 14.06.2007 DE 102007028039
(71) Anmelder: Carl Freudenberg KG, 69469 Weinheim (DE)
(72) Erfinder: Schaadt, Annette, 86159 Augsburg (DE); Peczynski, Mirko, 94526 Metten (DE); Schedl, Wolfgang, 86152 Augsburg (DE); Hunger, Marc, 86151 Augsburg (DE)

(57) **Zusammenfassung**

Es soll ein Reinigungsartikel, umfassend einen Vliesstoff mit mechanisch stabiler dreidimensionaler Struktur, der gleichzeitig besonders leicht, weich, saug- und aufnahmefähig für Feuchtigkeit, Nässe und Schmutz sowie besonders gut waschbar ist, bereitgestellt werden.

Dazu umfasst der Reinigungsartikel einen Vliesstoff mit einem Flächengewicht von 50 bis 300 g/m² und einer Dicke von 2 bis 6 mm, wobei der Vliesstoff mechanisch, chemisch und/oder thermisch verfestigt ist, wobei dessen Höchstzugkraft in mindestens einer Richtung 100 bis 300 N/50 mm beträgt, dessen Höchstzugkraftdehnung in Längsrichtung höchstens 35% und in Quemchtung höchstens 70% beträgt, dessen Wasseraufnahmefähigkeit das 2-bis 10-Fache des eigenen \AiesstoffgevAchts beträgt, wobei der Vliesstoff weich ist, eine dreidimensionale Struktur mit Poren bzw. Kavitäten aufweist und wobei der Vliesstoff
- als bindergebundener Vliesstoff 20 bis 90 Gew.-% Baumwoll- und/oder Viskosefasern, 5 bis 50 Gew.-% Kunststofffasern, insbesondere Polyesterfasern, und/oder 5 bis 35 Gew.-% Polyolefinfasem, insbesondere Polypropytenfasern, umfasst und der mit 2 bis 20 Gew.-% eines polymeren Binders ausgewählt aus Latex-Suspensionen imprägniert ist, dessen Feststoffanteil 1 bis 60 Gew.-%, bevorzugt 5 bis 20 Gew.-%, beträgt oder wobei der Vliesstoff
- als Bikompononenten-Vliesstoff 5 bis 50 Gew.-% Bikomponentenfasern, insbesondere Kern-Mantel-Fasem aus höher schmelzendem Kern und niedriger schmelzendem Mantel, mit Mantel-Fasern aus Polyester mit einem Anteil von 5 bis 30 Gew. °%, umfasst und Baumwoll- undloder Viskosefasern mit einem Anteil von 20 bis 90 Gew.-%, bevorzugt 50 bis 85 Gew.%, und/oder Polyolefinfasern, insbesondere Polypropylenfasern, mit einem Anteil von 5 bis 50 Gew.%, bevorzugt von 5 bis 20 Gew.-% oder wobei der Vliesstoff
- als Saridwich-Vliesstoff mindestens dreilagig aufgebaut ist, wobei zumindest eine innere Lage gleich oder zu mehr als 50 Gew.% aus Kunststofffasem aufgebaut ist und die äußeren Lagen zu mehr als 50 Gew.% aus Baumwoll- und/oder Viskosefasern aufgebaut sind und wobei der Sandwich-Vliesstoff gegebenenfalls mit 2 bis 20 Gew.-% eines polymeren Binders ausgewählt aus Latex-Suspensionen imprägniert ist, dessen Feststoffanteil 1 bis 60 Gew.-%, bevorzugt 5 bis 20 Gew.%, beträgt

## Beschreibung

### Technisches Gebiet

Die vorliegende Erfindung betriftt einen Reinigungsartikel, insbesondere für eine Feucht- oder Nassreinigung, umfassend einen Vliesstoff und dessen Verwendung zur Reinigung der menschlichen Haut oder von Oberflächen im Haushalt oder in der Industrie.

Vliesstoffe, die für Flüssigkeiten besonders saugfähig sind, sind insbesondere im Bereich der Verwendung als Reinigungsartikel für eine Feucht- oder Nassreinigung besonders erwünscht.

Zudem sollen die als Reinigungsartikel eingesetzten Vliesstoffe auch besonders strapazierfähig sein und den mechanischen Beanspruchungen beim Gebrauch und beim anschließenden Reinigen, beispielsweise unter den Bedingungen einer Maschinenwäsche, standhalten.

Des Weiteren sollen die Reinigungsartikel besonders gut zur Schmutzaufnahme geeignet sein.

Eine leichte Reinigung und Auswaschbarkeit der Reinigungsartikel ist insbesondere aus hygienischen Gesichtspunkten im Bereich vielfach verwendbarer Reinigungsartikel von besonderer Bedeutung.

Eine weiche Ausgestaltung eines Vliesstoffes sorgt für eine angenehme Haptik und für eine sanfte Reinigung empfindlicher Oberflächen, wie beispielsweise der menschlichen Haut oder auch kratzempfindlicher Oberflächen von Gegenständen und ist daher ebenfalls wünschenswert.

### Darstellung der Erfindung

Aufgabe der vorliegenden Erfindung ist die Bereitstellung von Vliesstoff-Reinigungsartikeln mit mechanisch stabiler dreidimensionaler (3D) Struktur, die gleichzeitig besonders weich, saug- und aufnahmefähig für Feuchtigkeit, Nässe und Schmutz sowie besonders gut waschbar sind.

Die Lösung der gestellten Aufgabe wird mit den Merkmalen des Anspruchs 1 erreicht

Die erfindungsgemäßen Reinigungsartikel umfassen Vliesstoffe, die mittels mechanischer, thermischer und/oder chemischer Bindung verfestigt sind.

Zudem weist der Vliesstoff eine dreidimensionale Struktur mit Poren bzw. Kavitäten zur Schmutzaufnahme auf.

Der Vliesstoff umfasst als eine Alternative 20 bis 90 Gew.-% Baumwoll- und/oder Viskosefasern, 5 bis 50 Gew.-% Kunststofffasern, insbesondere Polyesterfasern, und/oder 5 bis 35 Gew.-% Polyolefinfasem, insbesondere Polypropylenfasern, und ist mit 2 bis 20 Gew.-% eines polymeren Binders ausgewählt aus Latex-Suspensionen imprägniert, dessen Feststoffanteil 1 bis 60 Gew.-% beträgt. Dieser bindergebundene Vliesstoff lässt sich thermisch nachverformen und weist eine besonders hohe Strukturstabilität auf sowie eine ausreichende Weichheit, eine besondere Wasseraufnahmefähigkeit und Waschbarkeit.

Alternativ zu dem vorgenannten bindergebundenen Vliesstoff umfasst der Bikomponenten-Vliesstoff 5 bis 50 Gew.-% Bikomponentenfasem, insbesondere Kern-Mantel-Fasern aus höher schmelzendem Kern und niedriger schmelzendem Mantel, mit Mantel-Fasern aus Polyester mit einem Anteil von 5 bis 30 Gew.-% und Baumwoll- und/oder Viskosefasern mit einem Anteil von 20 bis 90 Gew.-%, bevorzugt 50 bis 85 Gew.-%, und/oder Polyolefinfasern, bevorzugt Polypropylenfasem,mit einem Anteil von 5 bis 20, bevorzugt 5 bis 50 Gew.-%.

Als weitere Alternative zu dem bindergebundenen Vliesstoff oder dem Bikomponenten-Vliesstoff ist ein Sandwich-Vliesstoff vorgesehen, der mindestens dreilagig aufgebaut ist, wobei zumindest eine innere Lage zu gleich oder zu mehr als 50 Gew.-% aus Kunststofffasern aufgebaut ist und wobei die äußeren Lagen zu mehr als 50 Gew.-% aus Baumwoll- und/oder Viskosefasern aufgebaut sind und wobei der Sandwich-Vliesstoff gegebenenfalls mit 2 bis 20 Gew.-% eines polymeren Binders ausgewählt aus Latex-Suspensionen imprägniert ist, dessen Feststoffanteil 1 bis 60 Gew.-% beträgt, (bindergebundener sandwich-Vliesstoff). Die Sandwich Varianten sind besonders strukturstabil durch den hohen Anteil an Kunststofffasern in der Mittellage, und sie sind besonders weich und saugfähig durch den hohen Naturfaseranteil.

Der Vliesstoff des Reinigungsartikels aller drei Alternativen weist eine Höchstzugkraft in mindestens einer Richtung von 100 bis 300 N/50 mm auf, eine Höchstzugkraftdehnung in Längsrichtung von höchstens 35% und in Querrichtung von höchstens 70% sowie eine Wasseraufnahmefähigkeit um das 2- bis 10-Fache des eigenen Vliesstoffgewichts.

Der Vliesstoff des Reinigungsartikels aller drei Alternativen ist ferner besonders weich, das heißt, er weist eine geringe Fasersteifigkeit auf, und sorgt daher für eine angenehme Haptik und ist für eine sanfte Reinigung empfindlicher Oberflächen besonders geeignet.

In bevorzugter Ausgestaltung des Reinigungsartikels besteht der Vliesstoff aus Stapelfasern, die trockengelegt sind, insbesondere mittels Krempeln, und mechanisch verfestigt sind, insbesondere mittels Nadeln, und thermisch verfestigt und/oder mittels eines Binders chemisch verfestigt sind sowie strukturiert sind durch Tiefziehen in einem Prägekalander.

Thermisch verfestigte Vliesstoffe sind grundsätzlich besonders weich. Es können sich jedoch Fasern aus der Oberfläche bei der Anwendung herauslösen.

Bindergebundene Vliesstoffe sind grundsätzlich etwas weniger weich, sind dagegen jedoch sehr stabil gegenüber dem Herauslösen von Fasern aus der Oberfläche bei der Anwendung.

Eine nachträgliche Prägung durch Tiefziehen in einem Prägekalander ermöglicht die Bildung einer stabilen dreidimensionalen Struktur mit sogenannten Pick-up-Poren bzw. Kavitäten, die besonders zur Schmutzaufnahme geeignet sind, unter gleichzeitigem Erhalt der Weichheit und Saugfähigkeit des Vliesstoffes.

Die Kombination der genannten Materialzusammensetzungen zusammen mit dem Verfahren führt zu einem Vliesstoff, der durch eine gleichmäßige Verformung in allen Richtungen eine gleichmäßige Struktur aufweist, in der weder in den Bergen noch in den Tälern der Struktur platte, geschmolzene Teilgebiete entstehen. Es findet folglich eine thermische Verformung ohne Plastffizierung statt.

Bei der Wahl einer chemischen Verfestigung mittels eines Binders ist der Binder vorteilhafterweise ausgewählt aus Nitrilbutadienkautschuk (NBR) Latex-Suspensionen.

Die Kunststofffasern für eine innere Lage eines mindestens dreilagig aufgebauten Sandwich-Vliesstoffes sind ausgewählt aus thermoplastischen Kunststoffen, wie Polyolefinen, Polyestern, Polyacrylnitrilen, Polyamiden, Polyimiden, Polycarbonaten und/oder deren Copolymeren und/oder deren Gemischen, bevorzugt ausgewählt aus Polypropylen und Polyethylenterephthalat.

In bevorzugter Ausgestaltung des Reinigungsartikels umfasst der bindergebundene Vliesstoff 60 bis 65 Gew.-% Viskosefasern, 20 bis 30 Gew.-% Polyethylenterephthalatfasern und/oder 10 bis 20 Gew.-% Polypropylenfasern und ist mit Nitrilbutadienkautschuk (NBR) Latex als Binder imprägniert, dessen Feststoffanteil 10 bis 20 Gew.-% beträgt.

Alternativ zu dem bindergebundenen Vliesstoff umfasst der Bikomponenten-Vliesstoff vorteilhafterweise Bikomponentenfasern aus Polyethylenterephthalat mit einem Anteil von 15 bis 20 Gew.-% und ferner Viskosefasern mit einem Anteil von 60 bis 70 Gew.-% und Polypropylenfasern mit einem Anteil von 10 bis 20 Gew.-%.

Der mindestens dreilagig aufgebaute Sandwich-Vliesstoff ist, weist bevorzugt zumindest eine innere Lage mit 30 bis 70 Gew.-% Polyolefinfasem, insbesondere. Polypropylenfasern, und 70 bis 30 Gew.-% Polyesterfasern, insbesondere Polyethylenterephthalatfasern, auf oder er weist bevorzugt als zumindest eine innere Lage 30 bis 40 Gew.-% Polyolefinfasern, insbesondere Polypropylenfasern, und zu 25 bis 50 Gew.-% Polyesterfasern, insbesondere Polyethylenterephthalatfasern, sowie 20 bis 30 Gew.-% Viskosefasern auf. Die äußeren Lagen sind beim mindestens dreilagig aufgebauten Sandwich-Vliesstoff bevorzugt zu gleich oder mehr als 85 Gew.-% aus Viskosefasern und zu gleich oder weniger als 15 Gew.-% aus Polyolefinfasern, insbesondere Polypropylenfasern, aufgebaut

Die erfindungsgemäßen Reinigungsartikel sind bevorzugt waschstabil bei Temperaturen bis 60°C.

Aufgrund ihrer vorgenannten Eigenschaften sind die erfindungsgemäßen Reinigungsartikel für die Feucht- oder Nassreinigung bevorzugt und vorteilhafterweise zur Reinigung der menschlichen Haut oder selbst von empfindlichen Oberflächen im Haushalt oder in der Industrie verwendbar. Die erfindungsgemäßen Reinigungsartikel finden daher vorteilhafterweise als Reinigungstuch oder in einem Wischmop Verwendung.

### Ausführung der Erfindung

Der Gegenstand der Erfindung wir anhand eines Beispiels näher erläutert.

Als Fasern werden Stapelfasern mit im Wesentlichen runden Querschnitt eingesetzt.

Die Vliesstoffherstellung erfolgt Ober ein Trockeniegungsverfahren, insbesondere mittels Krempeln, und über eine mechanische Verfestigung, insbesondere über einen Vernadelungsschritt. Anschließend erfolgt eine Imprägnierung und Trocknung oder allein eine thermische Verfestigung.

So werden Vliesstoffe mit einem Gewicht von 50 bis 300 g/m² hergestellt mit einer Höchstzugkraft in mindestens einer Richtung von 100 bis 300 N/ 50 mm. Die Wasseraufnahmefähigkeit beträgt das 2- bis 10-Fache des eigenen Vliesstoffgewichts.

Der circa 0,7 bis 1,5 mm dicke Vliesstoff wird danach in einem weiteren Prozess strukturiert. Hierzu wird der Vliesstoff mittels Strahlungswärme kontaktlos auf Temperaturen zwischen 100 und 24.0°C erhitzt. Die Strukturierung erfolgt kontinuierlich durch Tiefziehen in einem temperierten Prägekalander. Das Material kann dabei seitlich gehalten werden. Die hierbei entstehende Struktur des Vliesstoffes hat eine Dicke von 2 bis 6 mm.

Die derart hergestellte 3-dimensionale Struktur hat sogenannte Pick-up-Poren bzw. Kavitäten, die besonders zur Schmutzaufnahme geeignet sind. Ferner ist die dreidimensionale Struktur besonders mechanisch stabil.

Das Gewicht, die Wasseraufnahmefähigkeit und die Zugkräfte werden durch die Strukturierung nicht wesentlich verändert und auch die Weichheit des Vliesstoffes bleibt erhalten.

### Herstellung der bindergebundenen Vliesstoffe;

Eine Stapetfasermischung aus Viskose- und/oder Baumwollfasern und Synthetikfasern, wobei der Synthetikanteil 10 bis 40 Gew.-%, bevorzugt 30 bis 40 Gew.-%, beträgt, wird mittels ein bis drei Krempeln aufgelöst, und ein Faserflor wird gebildet. Der nach dem Krempeln angeordnete Kreuzleger nimmt den von den Krempeln gebildeten Flor ab und schichtet diesen in mehreren Lagen zu einem Vlies übereinander.

Die erste Verfestigung des Vlieses zu einem Nadelvliesstoff erfolgt mit ein bis drei Nadelmaschinen von beiden Seiten des Vlieses.

Nach der Vemadelung wird der Vliesstoff zur weiteren Verfestigung imprägniert. Dazu erfolgt eine Applizierung mit einer verschäumten Bindeflüssigkeit. Die Bindeflüssigkeit enthält NBR-Latex und gegebenenfalls Hilfsmittel. Der Schaum wird in dem Zwickel eines Foulards dosiert, und an einem Walzenpaar wird die überschüssige Flotte abgequetscht. Somit werden bei einer Bindeflüssigkeit, die circa 6 Gew.-% NBR-Latex enthält, circa 15 % Feststoff bezogen auf das Gesamtgewicht aufgetragen.

Im Anschluss an die Imprägnierung erfolgt eine Trocknung des Vliesstoffes und eine Vernetzung des Bindemittels. Für die Trocknung und Vernetzung werden Schwebetrockner und/oder Zylindertrockner eingesetzt. Dabei werden Temperaturen von 160 bis 200°C verwendet.

Der Vliesstoff wird daraufhin in einem weiteren Prozess, wie oben beschrieben, strukturiert.

Es ist dabei überraschend, dass sich ein bindergebundener Vliesstoff, dessen Binder kein thermoplastisches Material ist, überhaupt thermisch stabil nachverformen lässt.

So wird ein Vliesstoff mit einer Dicke von 2 bis 6 mm mit einem Gewicht von etwas mehr als 100 g/m² hergestellt mit einer Höchstzugkraft in mindestens einer Richtung von mindestens 100 N/ 50 mm. Durch den Feststoffgehalt an Binder wird eine besondere Strukturstabilität bewirkt, die dennoch gleichzeitig noch eine ausreichende Weichheit besitzt. Die Wasseraufnahmefähigkeit beträgt das 6- bis 9-Fache des eigenen Vliesstoffgewichts.

Für den bindergebundenen Vliesstoff gemäß den Daten der Tabelle wurde eine Fasermischung aus
61 Gew.-% Viskose,
24 Gew.-% Polyethylenterephthalat (PET),
15 Gew.-% Polypropylen (PP),
der mit 6 Gew.-% Nitrilbutadienkautschuk (NBR) Latex imprägniert ist, dessen Feststoffanteil 15 Gew.-% beträgt, eingesetzt.

Die Imprägnierung enthält des Weiteren abhängig von der gewünschten Farbgebung und dem Bedarf gegebenenfalls noch zumindest einen Farbstoff.

### Herstellung der Bikomponenten-Vliesstoffe:

Eine Stapelfasermischungaus Viskose- und/oder Baumwollfasern und Synthetikfasern sowie Kern-Mantel-Bikomponentenfasern aus höher schmelzendem Kern und niedriger schmelzendem Mantel, wird mittels ein bis drei Krempeln aufgelöst, und ein Faserflor wird gebildet. Der nach dem Krempeln angeordnete Kreuzleger nimmt den von den Krempeln gebildeten Flor ab und schichtet diesen in mehreren Lagen zu einem Vlies übereinander.

Die erste Verfestigung des Vlieses zu einem Nadelvtiesstoff erfolgt mit ein bis drei Nadelmaschinen von beiden Seiten des Vlieses.

Nach den Nadelmaschinen erfolgt eine Thermofixierung des Vliesstoffes. Als erstes läuft er in eine Thermofusionierung. In den mit Heißluft beheizten Siebbandanlagen werden Temperaturen bis 200°C eingesetzt. Durch das Anschmelzen der Klebefasern wird so die Stabilität des Vliesstoffes erhöht. Nach der Thermofusionierung erfolgt ein sogenanntes Thermobonding bei dem eine thermische Verfestigung durch einen beheizten Kalander erfolgt. Durch den hohen Druck und Temperaturen bis 250°C im Kalanderspalt wird der Vliesstoff verdichtet, und die Fasern werden gebunden, insbesondere über die niedriger schmelzenden Mantel-Fasern.

Der Bikomponenten-Vliesstoff lässt sich thermisch nachverformen und wird in einem weiteren Prozess, wie oben beschrieben, strukturiert, wobei insbesondere die Kern- und Mantelfasem verformt werden.

So wird ein Vliesstoff mit einer Dicke von 2 bis 6 mm mit einem Gewicht von 100 bis 200 g/m² hergestellt mit einer Höchstzugkraft in mindestens einer Richtung von 100 bis 200 N/50 mm, der mithin eine besondere mechanische Strukturstabilität aufweist, und der zudem noch ausreichend weich ist Die Wasseraufnahmefähigkeit beträgt das 6- bis 9-Fache des eigenen Vliesstoffgewichts.

Für den Bikomponenten-Vliesstoff gemäß den Daten der Tabelle wurde eine Fasermischung aus
20 Gew.-% Kern-Mantel-Bikomponentenfasern
aus einem Polyethylenterephthalat-Kern mit einer Schmelztemperatur von 263°C und einem Co- Polyethylenterephthalat-Mantel mit einer Schmelztemperatur von 160°C,
65 Gew.-% Viskose und
15 Gew.-% Polypropylen (PP)
eingesetzt.

### Herstellung der Sandwich-Vliesstoffe:

Eine Stapelfasermischung für eine innere Lage aus mehr als 50 Gew.-% Kunststofffasern und eine Stapelfasermischung für die äußeren Lagen aus mehr als 50 Gew.-% Viskose- und/oder Baumwollfasern wird für einen dreilagigen Sandwich-Vliesstoff mittels drei Krempeln aufgelöst, und ein Faserflor wird gebildet. Der nach dem Krempeln angeordnete Kreuzleger nimmt den von den Krempeln gebildeten Flor ab und schichtet diesen in mehreren Lagen zu einem Vlies übereinander.
Die Krempeln eins und drei sind für die Außenlagen des Vliesstoffes, Die Krempel zwei ist für die Mittellage des Vliesstoffes verantwortlich.

Die erste Verfestigung des Vlieses zu einem Nadelvliesstoff erfolgt mit ein bis drei Nadelmaschinen von beiden Seiten des Vlieses.

Nach den Nadelmaschinen erfolgt eine Thermofixierung des Vliesstoffes. Als erstes läuft er in eine Thermofusionierung. In den mit Heißluft beheizten Siebbandanlagen werden Temperaturen bis 200°C eingesetzt. Durch das Anschmelzen der Klebefasem wird so die Stabilität des Vliesstoffes erhöht. Nach der Thermofusionierung erfolgt ein sogenanntes Thermobonding bei dem eine thermische Verfestigung durch einen beheizten Kalander erfolgt. Durch den hohen Druck und Temperaturen bis 250°C im Kalanderspalt wird der Vliesstoff verdichtet, und die Fasern werden gebunden, Hierdurch erhält der Vliesstoff im Wesentlichen seine Festigkeit.

Der Vliesstoff wird daraufhin in einem weiteren Prozess, wie oben beschrieben, strukturiert.

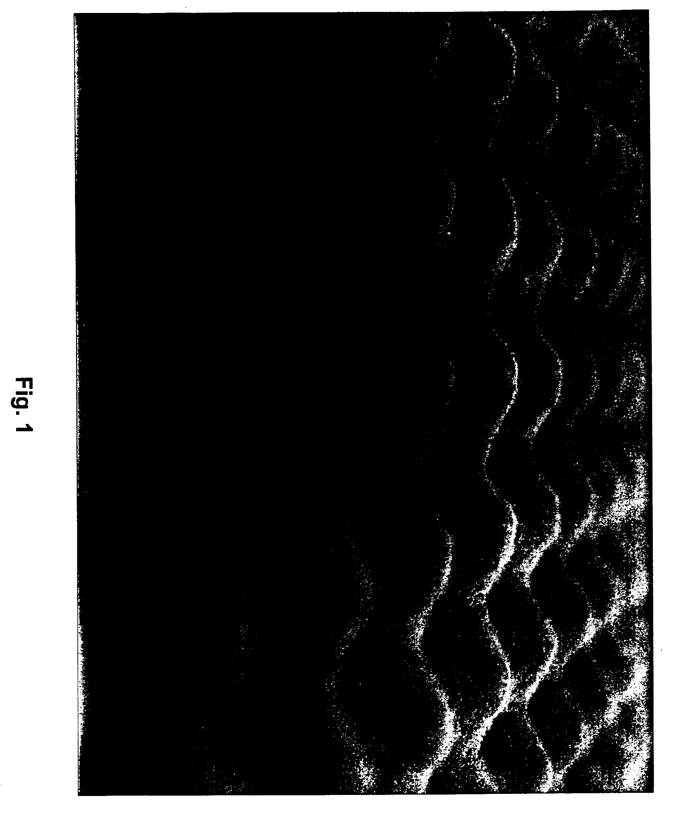

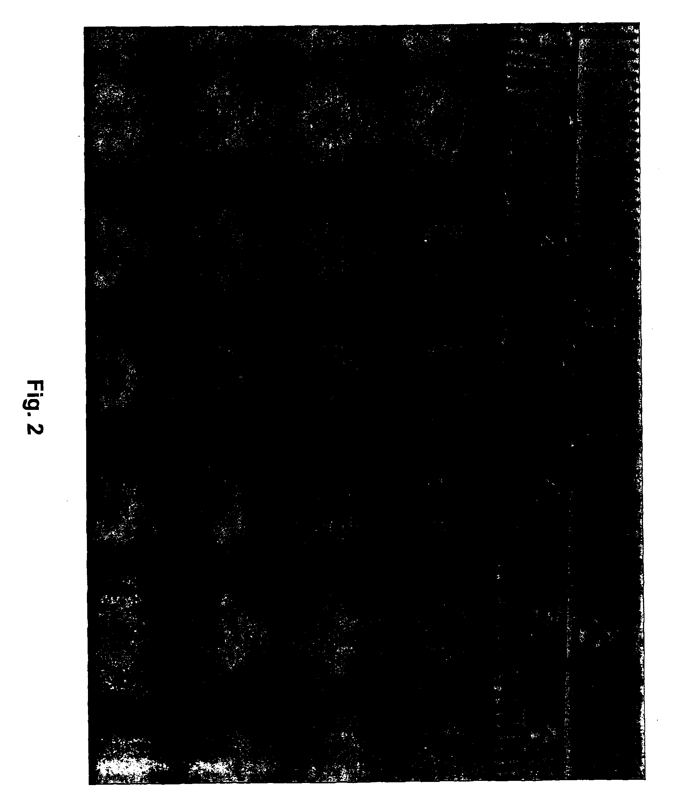

Zur Veranschaulichung der Struktur des Vliesstoffes dienen die Figuren 1 und 2. Die darin gezeigten Fotos sind mit einer Fotokamera Canon Powershot G2 (4 Mega Pixel) aufgenommen worden.

Figur 1 zeigt eine seitliche Aufsicht und Figur 2 zeigt eine direkte Aufsicht der Oberflächenstruktur des Sandwich-Vliesstoffes.

Die besondere Kombination der stofflichen und strukturellen Zusammensetzung führt zu einer besonderen mechanischen Stabilität des Produkts unter Beibehaltung der Weichheit und der besonders guten Wasseraufnahmefähigkeit.

So wird ein Vliesstoff mit einer Dicke von 2 bis 6 mm mit einem Gewicht von ungefähr 200 g/m² hergestellt mit einer Höchstzugkraft in mindestens einer Richtung von ungefähr 200 bis 250 N/ 50 mm. Dabei weist das Flächengebilde für eine Innenlage ein Gewicht von 40 bis 60 g/m² auf und je Außenlage ein Gewicht von 40 bis 80 g/m² auf. Die Wasseraufnahmefähigkeit beträgt das 3-bis 7-Fache des eigenen Vliesstoffgewichts.

Für den dreilagigen Sandwich-Vliesstoff gemäß den Daten der Tabelle wurde eine Fasermischung aus
15 Gew.-% Polypropylen (PP) und 85 Gew.-% Viskose für jede Außenlage und aus 50 Gew.-% Polypropylen (PP) und 50 Gew.-% Polyethylenterephthalat (PET) für die Innenlage eingesetzt.

**Tabelle**

| | Bindergebundener Vliesstoff (Mittelwerte) | Bikomponenten-Vliesstoff (Mittelwerte) | Sandwich-Vliesstoff (Mittelwerte) |
|---|---|---|---|
| Dicke [mm] | 3,6 | 5,0 | 5 |
| Gewicht [g/m²] | 105 | 170 | 208 |
| Wasseraufnahme trocken [%] | 860 | 932 | 640 |
| Wasseraufnahme feucht [%] | 646 | 627 | 348 |
| Höchstzugkraft längs [N/50 mm] | 105 | 176 | 250 |
| Höchstzugkraft quer [N/50 mm] | 100 | 131 | 204 |
| Höchstzugkraft-dehnung längs [%] | 30 | 29 | 32 |
| Höchstzugkraft-dehnung quer [%] | 60 | 58 | 42 |
| Krumpf längs [%] angelehnt an 150 3759 | 9 | 4 | 4 |
| Krumpf quer [%] angelehnt an ISO 3759 | 5 | 3 | 2 |
| Dicke nach Wäsche bei 60°C und Trocknen [mm] angelehnt an ISO 3759 | 1,8 | 4 | 3 |

Von dem jeweiligen Vliesstoff werden jeweils Proben ausgestanzt (Größe DIN A4. 210 x 297 mm), und die Proben werden in einer Miele Waschmaschine (Miele Novotronic ® W 725 oder Miele Softtronic Vitality ® W 400) bei 60°C (Programm: Koch-/Buntwäsche, Schleudern: 1200 Upm) mit Persil ® gewaschen und anschließend in einem Miele Wäschetrockner (Programm: extra trocken) getrocknet Nach dem Trocknen wird die Länge und Breite der Proben gemessen.

Wie die vorgenannten Daten zeigen, sind die erfindungsgemäßen Reinigungsartikel waschstabil bei Temperaturen bis 60°C.

## Patentansprüche

1. Reinigungsartikel, insbesondere für eine Feucht- oder Nassreinigung, umfassend einen Vliesstoff mit mechanisch stabiler dreidimensionaler Struktur, einem Flächengewicht von 50 bis 300 g/m² und einer Dicke von 2 bis 6 mm, wobei der Vliesstoff mechanisch, chemisch und/oder thermisch verfestigt ist, wobei dessen Höchstzugkraft in mindestens einer Richtung 100 bis 300 N/50 mm beträgt, dessen Höchstzugkraftdehnung in Längsrichtung höchstens 35% und in Querrichtung höchstens 70% beträgt, dessen Wasseraufnahmefähigkeit das 2- bis 10-Fache des eigenen Vliesstoffgewichts beträgt, wobei der Vliesstoff weich ist, eine dreidimensionale Struktur mit Poren bzw. Kavitäten aufweist und wobei der Vliesstoff
- als bindergebundener Vliesstoff 20 bis 90 Gew.-% Baumwoll- und/oder Viskosefasern, 5 bis 50 Gew.-% Kunststofffasern, insbesondere Polyesterfasern, und/oder 5 bis 35 Gew.-% Polyolefinfasern, insbesondere Polypropylenfasern, umfasst und mit 2 bis 20 Gew.-% eines polymeren Binders ausgewählt aus Latex-Suspensionen imprägniert ist, dessen Feststoffanteil 1 bis 60 Gew.-%, bevorzugt 5 bis 20 Gew.-%, beträgt oder wobei der Vliesstoff
- als Bikompononenten-Vliesstoff 5 bis 50 Gew.-% Bikomponentenfasem, insbesondere Kern-Mantel-Fasern aus höher schmelzendem Kern und niedriger schmelzendem Mantel, mit Mantel-Fasern aus Polyester mit einem Anteil von 5 bis 30 Gew.-%, umfasst und Baumwoll- und/oder Viskosefasern mit einem Anteil von 20 bis 90 Gew.-%, bevorzugt 50 bis 85 Gew.-%, und/oder Polyolefinfasern, insbesondere Polypropylenfasern, mit einem Anteil von 5 bis 50 Gew.-%, bevorzugt von 5 bis 20 Gew.-% oder wobei der Vliesstoff
- als Sandwich-Vliesstoff mindestens dreilagig aufgebaut ist, wobei zumindest eine innere Lage gleich oder zu mehr als 50 Gew.-% aus Kunststofffasern aufgebaut ist und die äußeren Lagen zu mehr als 50 Gew.-% aus Baumwoll- und/oder Viskosefasern aufgebaut sind und wobei der Sandwich-Vliesstoff gegebenenfalls mit 2 bis 20 Gew.-% eines polymeren Binders ausgewählt aus Latex-Suspensionen imprägniert ist, dessen Feststoffanteil 1 bis 60 Gew.-%, bevorzugt 5 bis 20 Gew.-%, beträgt.

2. Reinigungsartikel, insbesondere für eine Feucht- oder Nassreinigung, nach Anspruch 1, bei dem der Vliesstoff aus Stapelfasern trockengelegt ist, insbesondere mittels Krempeln, und mechanisch verfestigt ist, insbesondere mittels Nadeln, und thermisch verfestigt ist und/oder mittels eines Binders chemisch verfestigt ist sowie dreidimensional strukturiert ist durch Tiefziehen in einem Prägekalander.

3. Reinigungsartikel, insbesondere für eine Feucht- oder Nassreinigung, nach Anspruch 1 oder 2, bei dem der Binder beim bindergebundenen Vliesstoff ausgewählt ist aus Nitrilbutadienkautschuk (NBR) Latex-Suspensionen und/oder bei dem die Kunststofffasern beim Sandwich-Vliesstoff ausgewählt sind aus Thermoplasten, insbesondere aus Polyolefinen, Polyestern, Polyacrylnitrilen, Polyamiden, Polyimiden, Polycarbonaten und/oder deren Copolymeren und/oder deren Gemischen, bevorzugt ausgewählt aus Polypropylen und Polyethylenterephthalat.

4. Reinigungsartikel, insbesondere für eine Feucht- oder Nassreinigung, nach einem der vorhergehenden Ansprüche, wobei
- der bindergebundene Vliesstoff 60 bis 65 Gew.-% Viskosefasern, 20 bis 30 Gew.-% Polyethylenterephthalatfasern und/oder 10 bis 20 Gew.-% Polypropylenfasern umfasst und mit NBR Latex als Binder imprägniert ist, dessen Feststoffanteil 10 bis 20 Gew.-% beträgt, oder
- der Bikomponenten-Vliesstoff Bikomponentenfasern aus Polyethylenterephthalat mit einem Anteil von 15 bis 20 Gew.-% umfasst und Viskosefasern mit einem Anteil von 60 bis 70 Gew.-% und Polypropylenfasern mit einem Anteil von 10 bis 20 Gew.-% umfasst oder
- der Sandwich-Vliesstoff mindestens dreilagig aufgebaut ist,
wobei zumindest eine innere Lage zu 30 bis 70 Gew.-% aus Polyolefinfasern, insbesondere Polypropylenfasern, und zu 70 bis 30 Gew.-% aus Polyesterfasern, insbesondere Polyethylenterephthalatfasern, aufgebaut ist oder
wobei zumindest eine innere Lage zu 30 bis 40 Gew.-% aus Polyolefinfasern, insbesondere Polypropylenfasern, und zu 25 bis 50 Gew.-% aus Polyesterfasern, insbesondere Polyethylenterephthalatfasern, und zu 20 bis 30 Gew.-% aus Viskosefasern aufgebaut ist und
wobei die äußeren Lagen zu gleich oder mehr als 85 Gew.-% aus Viskosefasern und zu gleich oder weniger als 15 Gew.-% aus Polyolefinfasern, insbesondere Polypropylenfasern, aufgebaut sind.

5. Reinigungsartikel, insbesondere für eine Feucht- oder Nassreinigung, nach einem der vorhergehenden Ansprüche, der waschstabil bei Temperaturen bis 60°C ist.

6. Verwendung des Reinigungsartikels, insbesondere für eine Feucht- oder Nassreinigung, nach einem der vorhergehenden Ansprüche zur Reinigung der menschlichen Haut oder von Oberflächen im Haushalt oder in der Industrie.

7. Verwendung des Reinigungsartikels, insbesondere für eine Feucht- oder Nassreinigung, nach einem der vorhergehenden Ansprüche als Reinigungstuch oder in einem Wischmop.
